# EUROPEAN PATENT APPLICATION

(11) **EP 1 512 423 A1**
(43) Date of publication of application: **09.03.2005**
(21) Application number: 04292081.9
(22) Date of filing: 24.08.2004
(51) Int. Cl.: A61M 5/145, A61M 5/00, A61M 5/168

(54) **Liquid injector in which a device for detecting the pressure exerted upon the liquid syringe piston is not arranged on a movable part**

(30) Priority: 02.09.2003 JP 2003309960
(71) Applicant: Nemoto Kyorindo Co., Ltd., Tokyo (JP)
(72) Inventor: Tanaka, Masafumi, Nemoto Kyorindo Co., Ltd., Tokyo (JP)
(74) Representative: Vercaemer, Laurence

(57) **Abstract**

In a liquid injector, a piston is pressed by a piston-driving mechanism into a cylinder of a liquid syringe that is held by a cylinder-holding structure, and a stress detection means detects the stress of this pressure. The piston-driving mechanism is displaceably supported by the main body of the injector, and the stress detection means, which is fixed to this injector main body, detects the stress that acts upon the piston-driving mechanism. The configuration of this liquid injector is simple because the stress detection means is not arranged on the movable part of the piston-driving mechanism, and the liquid injector therefore exhibits improved productivity and reliability.

## Description

### Background of the Invention:

### 1. Field of the Invention:

The present invention relates to a liquid injector for injecting liquid into a patient, and more particularly to a liquid injector in which the piston of a liquid syringe for injecting liquid into a patient is pressed into a cylinder.

### 2. Description of the Related Art:

Diagnostic imaging apparatuses for producing sectional images, i.e., tomographic images of a patient, currently include CT (Computed Tomography) scanners, MRI (Magnetic Resonance Imaging) devices, and PET (Positron Emission Tomography) devices; and diagnostic apparatuses for taking images of blood vessels, i.e., tomographic images of a patient, include CT angio devices and MRA (MR Angio) devices.

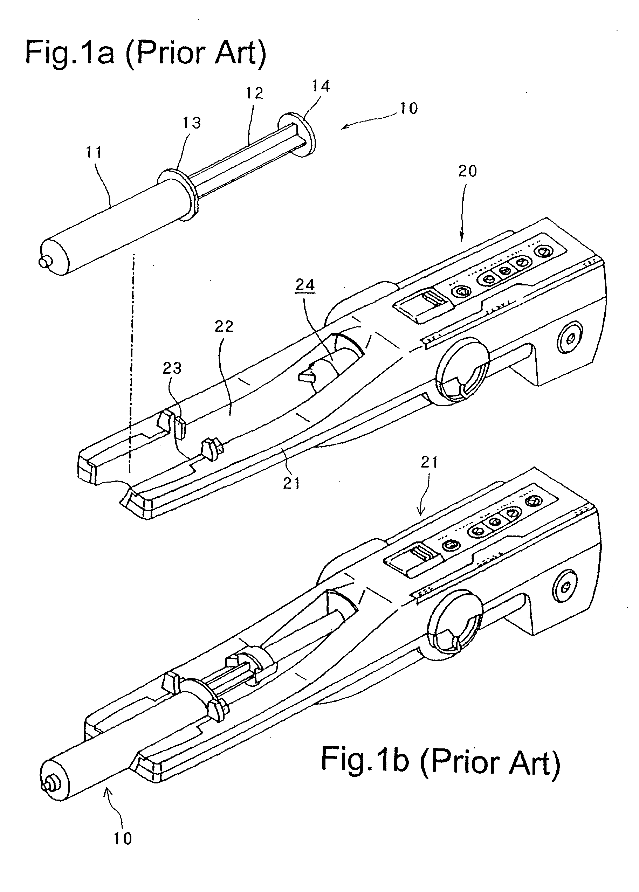

When using one of the above-described devices, the patient is injected with a liquid such as a contrast medium or a physiological saline solution, and liquid injectors for performing these injections automatically are now coming into practical use. Explanation next regards a prior-art example of such a liquid injector with reference to FIGs. 1 to 3.

First, as shown in FIG. 1, liquid syringe 10 is made up of one cylinder 11 and one piston 12, piston 12 being freely slidably inserted into cylinder 11. Cylinder flange 13 is formed around the outer trailing end of cylinder 11, and piston flange 14 is formed around the outer trailing end of piston 12.

The liquid injector (not shown) includes injector head 20, and concave portion 22 into which cylinder 11 of liquid syringe 10 is arranged is formed on the upper surface of injector main body 21 of this injector head 20. Cylinder holding structure 23 is formed in the forward portion of this concave portion 22 for holding cylinder flange 13 in a state that allows free insertion or removal, and piston-driving mechanism 24 is disposed in the rearward portion of concave portion 22 for holding piston 12 and causing piston 12 to slide.

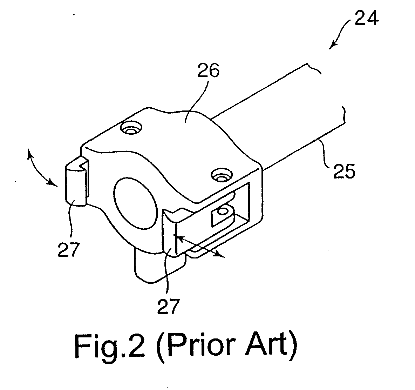

More specifically, as shown in FIG. 2, piston-driving mechanism 24 includes slide rod 25 that freely slides forward and backward, and piston-pressing member 26 for applying forward pressure against piston 12 is formed as a unit in the forward end of this slide rod 25.

This piston-pressing member 26 is furnished on the left and right sides with a pair of engaging catches 27 that can be freely opened or closed, and these catches 27 are elastically pressed in the closed direction by an elastic mechanism such as a coil spring. The tips of these engaging catches 27 are formed in a wedge shape, and these catches 27 engage respectively with the left and right portions of the front surface of piston flange 14 when piston-pressing member 26 is pressed against piston 12 from behind.

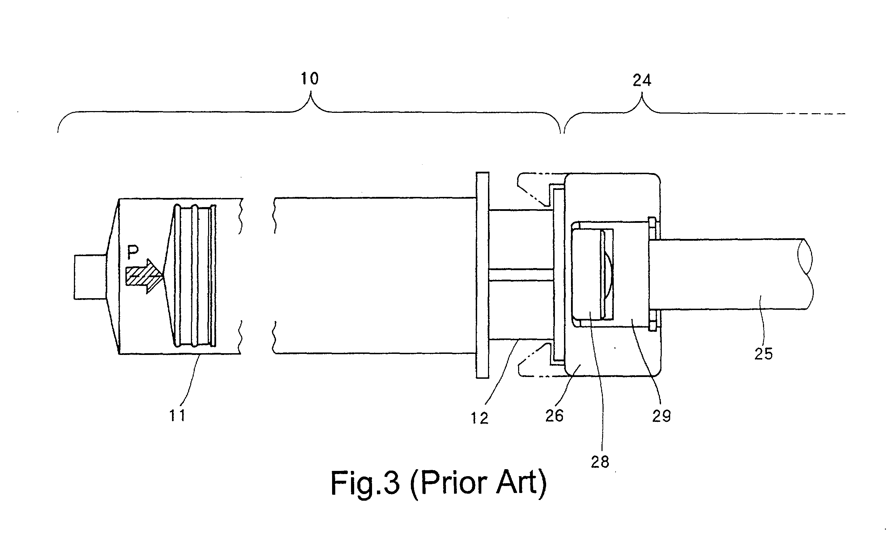

In addition, as shown in FIG. 3, piston-driving mechanism 24 includes load cell 28, and this load cell 28 generates an electrical signal that corresponds to the stress produced by the pressure of piston-driving mechanism 24 against piston 12 under the power of a drive motor (not shown).

More specifically, load cell 28 is installed in a freely slidable state in a concave portion in cell housing 29, this cell housing 29 is in turn installed in a freely slidable state in a concave portion in piston-pressing member 26 with load cell 28 contacting the bottom surface of this concave portion in piston-pressing member 26.

Cell housing 29 is installed at the leading end of slide rod 25 and cell housing 29 clasps piston 12 of liquid syringe 10, whereby the stress that is produced when piston-driving mechanism 24 exerts pressure upon piston 12 under the power of the drive motor acts upon load cell 28. Load cell 28 is connected to a computer unit (not shown), and this computer unit is connected to the drive motor of piston-driving mechanism 24.

Load cell 28 changes in electrical resistance corresponding to the degree of distortion, and this electrical resistance is acquired by the computer unit as an electrical signal. This computer unit detects the stress that is applied against piston 12 by piston-pressing member 26 based on the electrical signal of load cell 28 and implements feedback-control of piston-driving mechanism 24 in accordance with this stress.

In the above-described configuration, the liquid injector of this example of the prior art is capable of injecting a patient with liquid from liquid syringe 10, and is capable of injecting a patient with a liquid such as contrast medium for taking tomographic images with a CT scanner.

In such a case, in the initial state of the liquid injector, piston-driving mechanism 24 is disposed at the rearward end and liquid syringe 10 is loaded in concave portion 22, following which the start of injection of the liquid injector is instructed and piston-pressing member 26 of piston-driving mechanism 24 moves forward.

When forward-advancing piston-pressing member 26 presses against piston 12 from behind, the elastically closed pair of engaging catches 27 are pressed by piston flange 14 and are caused to gradually open. When piston-pressing member 26 moves still further forward, the pair of catches 27 engage with both sides of piston flange 14, whereby piston 12 is clasped by piston-pressing member 26.

In this state, piston 12 is pressed by piston-driving mechanism 24, and the patient is therefore injected with liquid from liquid syringe 10. At this time, piston-pressing member 26 that applies pressure against piston 12 also applies pressure against load cell 28, and the stress of pressure against piston 12 is thus detected by this load cell 28.

Piston-driving mechanism 24 is subjected to feedback control in accordance with this detected stress, and piston 12 is therefore pressed at an appropriate level of stress whereby, for example, piston-driving mechanism 24 is forcibly stopped when an abnormally high pressure is detected.

Patent applications for inventions of liquid injectors similar to the foregoing description have been previously filed by the present applicant and others. Reference may be made to, for example, Patent Documents 1 and 2:

### Patent Document 1:

Japanese Patent Laid-Open Publication No. 2002-11096 Patent Document 2:
Japanese Patent Laid-Open Publication No. 2002-102343

In the above-described liquid injector, pressure that presses piston 12 of liquid syringe 10 into cylinder 11 enables injection of liquid into a patient, and injecting a patient with liquid at an abnormally high pressure can be prevented by using load cell 28 to detect the pressure that is applied against piston 12.

However, the arrangement of load cell 28 in the interior of piston-pressing member 26 that undergoes a sliding movement together with piston 12 results in a complex structure of piston-pressing member 26 and therefore detracts from productivity. In particular, lead-out of the lines (not shown) that connect load cell 28 to the computer unit is complicated and prone to the occurrence of defects such as disconnections.

### Summary of the Invention:

The present invention was realized in view of the above-described problems and has as an object the provision of a liquid injector in which the pressure that presses the piston into the cylinder of a liquid syringe can be detected by a simple construction.

The liquid injector of the present invention is a liquid injector having a liquid syringe wherein a piston is freely slidably inserted into a cylinder that is filled with a liquid whereby the cylinder and piston are moved relative to each other to thus inject a patient with a liquid; wherein the liquid injector includes a cylinder-holding structure, a piston-driving mechanism, an injector main body, and a stress detection means.

The cylinder-holding structure holds the cylinder in a state that allows free installation and removal, and the piston-driving mechanism applies pressure to at least the piston and presses the piston into the cylinder. The cylinder-holding structure is fixed to the injector main body, and further, the piston-driving mechanism is supported by the injector main body in a state that allows free displacement of the piston-driving mechanism in the direction of sliding of the piston. The stress detection means is fixed to the injector main body and detects the stress that acts upon the piston-driving mechanism that applies pressure against the piston.

Accordingly, the liquid injector of the present invention enables the piston to be pressed by the piston-driving mechanism into the cylinder of a liquid syringe that is held by the cylinder-holding structure, and allows detection of the stress of this pressure by the stress detection means. The piston-driving mechanism is supported by the injector main body so as to allow free displacement, and the stress detection means that is fixed to this injector main body detects the stress that acts upon the piston-driving mechanism.

According the liquid injector of the present invention, when the piston is pressed by the piston-driving mechanism into the cylinder of the liquid syringe that is held by the cylinder-holding structure, the stress that acts about the piston-driving mechanism that is supported by the injector main body in a freely displaceable state is detected by a stress detection means that is fixed to the injector main body, and as a result, the stress detection means is not arranged on a movable portion of the piston-driving mechanism, and the configuration is therefore simple and exhibits excellent productivity and reliability.

Each of the various means described in the present invention may take any form that realizes its particular function, and for example, can be realized by dedicated hardware that exhibits a prescribed function, by a liquid injector that is provided with prescribed functions by a computer program, by prescribed functions that are realized by a liquid injector by means of a computer program, or by a combination of these means. In addition, each of the various means described in the present invention need not exist independently, but a plurality of means may be formed as a single component, a particular means may be a part of another means, or a part of a particular means may be duplicated with a part of another means.

The above and other objects, features, and advantages of the present invention will become apparent from the following description with reference to the accompanying drawings, which illustrate examples of the present invention.

### Brief Description of the Drawings:

FIG. 1 is a perspective view showing the state of loading or ejecting a liquid syringe in the injection head in an example of a liquid injector of the prior art.
FIG. 2 is a perspective view showing the outer appearance of the principle components of the piston-driving mechanism.
FIG. 3 is a schematic cross-sectional plan view showing the internal construction of the principle components of the piston-driving mechanism.
FIG. 4 is a schematic cross-sectional plan view showing the internal construction of the injection head of the liquid injector according to an embodiment of the present invention.
FIG. 5 is a perspective view showing the outer appearance of a liquid injection system.
FIG. 6 is a perspective view showing the outer appearance of liquid injector.
FIG. 7 is a perspective view showing the state of loading or ejecting a liquid syringe in the injection head.
FIG. 8 is a block diagram showing the circuit configuration of a liquid injection system.
FIG. 9 is a flow chart showing the processing operations of the liquid injector.
FIG. 10 is a flow chart showing the processing operations of an MRI device, which is an imaging diagnostic apparatus.
FIG. 11 is a perspective view showing a modification of an injection head.

### Detailed Description of the Preferred Embodiments:

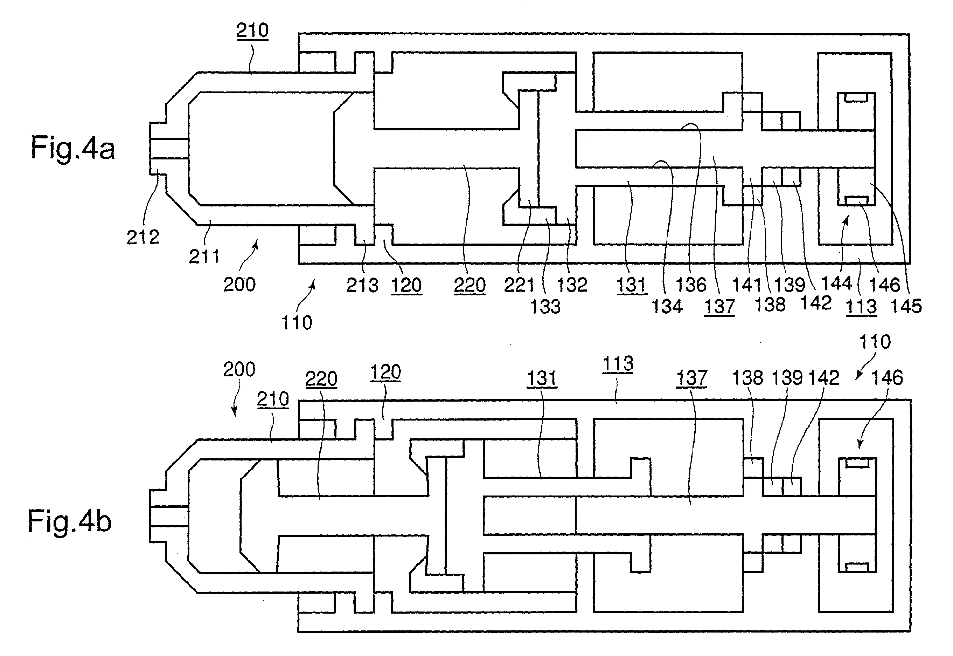

Explanation next regards an embodiment of the present invention with reference to FIGs. 4 to 10. As shown in the figures, explanation is given in the present embodiment by using the directions forward, rearward, upward, downward, leftward and rightward, but these directions are stipulated for the sake of convenience and for the purpose of simplifying the explanation of the relative relations of the various parts and do not limit the directions during fabrication or use when working the device of the present invention.

### Configuration of an Embodiment

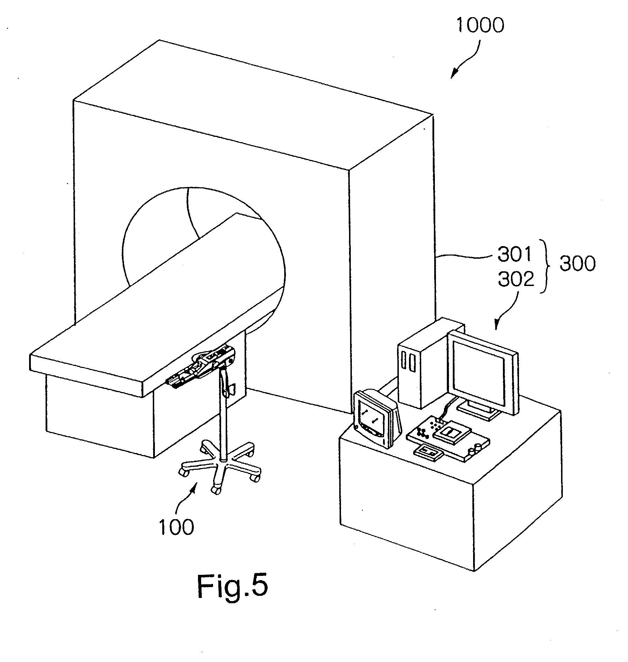

As shown in FlGs. 4 to 7, liquid injection system 1000 according to an embodiment of the present invention includes: liquid injector 100; liquid syringe 200; and MRI device 300, which is an imaging diagnostic device. Although described in greater detail hereinbelow, liquid injection system 1000 injects a contrast medium as the liquid into a patient (not shown).
As shown in FIG. 5, MRI device 300 includes tomographic unit 301, which is a construction for imaging, and imaging control unit 302; and tomographic unit 301 and imaging control unit 302 are cable-connected by communication network 303. Tomographic unit 301 takes tomographic images from the patient, and imaging control unit 302 controls the operations of tomographic unit 301.

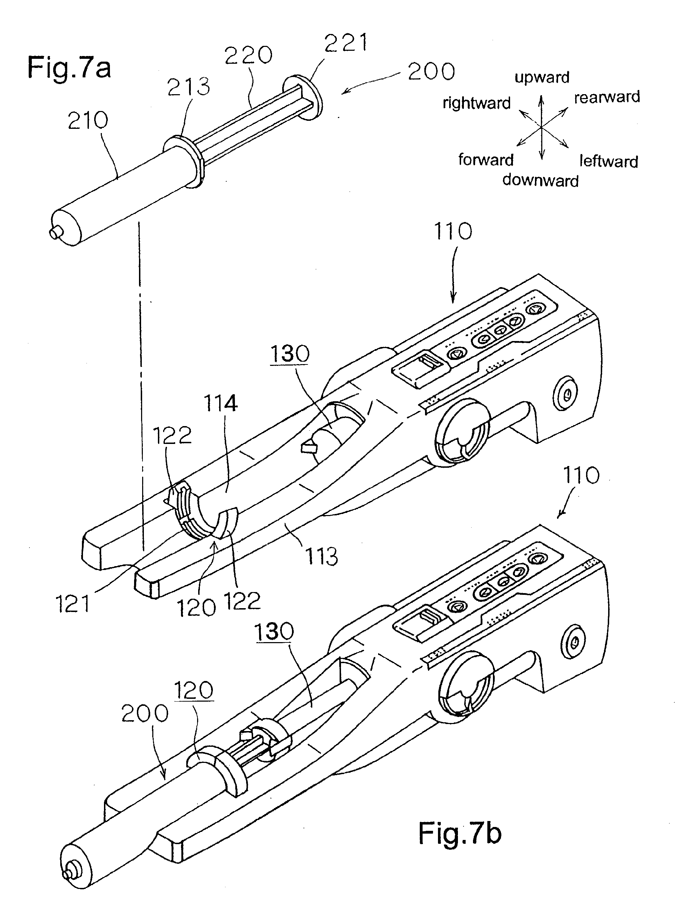

As shown in FIG. 4 and FIG. 7, liquid syringe 200 is made up of cylinder 210 and piston 220, piston 220 being inserted into cylinder 210 in a freely sliding state. Cylinder 210 includes cylindrical and hollow main body 211, and conduit 212 is formed in the surface of the blocked leading end of this main body 211.

The trailing end of main body 211 of cylinder 210 is open, and piston 220 is inserted into main body 211 from this opening. Cylinder flange 213 is formed on the outer circumference of the trailing end of cylinder 210, and piston flange 221 is formed around the outer circumference of the trailing end of piston 220.

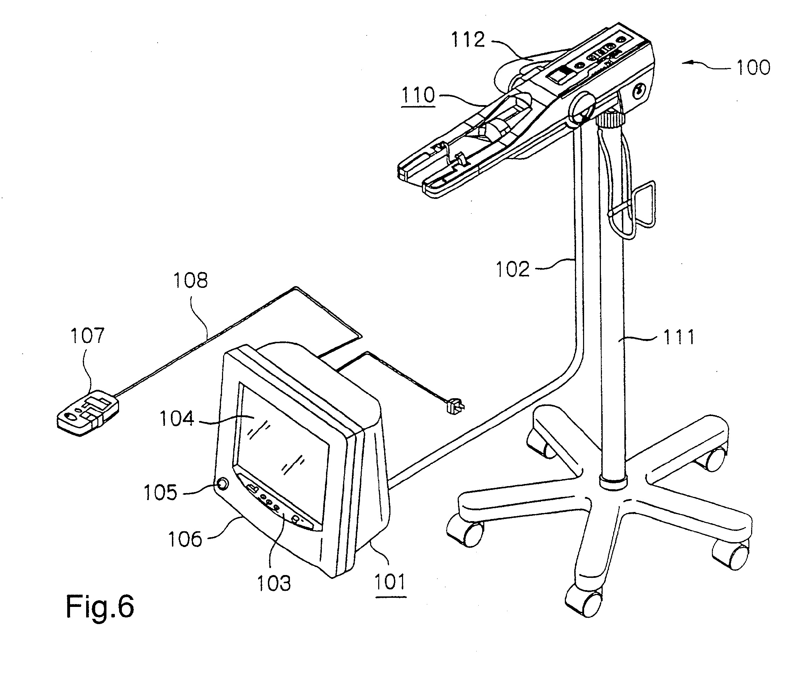

As shown in FIG. 6, in liquid injector 100 of the present embodiment, injection control unit 101 and injection head 110, which is the injector main body, are formed as separate units, and injection control unit 101 and injection head 110 are cable-connected by communication cable 102. Injection head 110 drives liquid syringe 200 that has been mounted to inject liquid into the patient, and injection control unit 101 controls the operation of injection head 110. For this purpose, injection control unit 101 incorporates computer unit 150 and is also cable-connected to imaging control unit 302 of MRI device 300 by means of communication network 304.

Injection control unit 101 includes components such as control panel 103; touch panel 104, which is a display panel; and speaker unit 105 on the front surface of its main unit housing 106; and separate controller unit 107 is cable-connected by means of connector 108.

Injection head 110 is mounted on the upper end of caster stand 111 by means of movable arm 112, and as shown in FIG. 7, concave portion 114 is formed on the upper surface of head main unit 113, which is the injector main body, this concave portion 114 being in the form of a semicylindrical channel for mounting liquid syringe 200 in a freely removable state. Cylinder-holding structure 120 for holding cylinder flange 213 of liquid syringe 200 in a freely installable or removable state is formed in the forward portion of this concave portion 114, and piston-driving mechanism 130 for holding piston flange 221 and causing piston flange 221 to slide is arranged rearward of this concave portion 114.

Cylinder-holding structure 120 includes stationary holding member 121 of one-third arc form that is arranged in the base for holding the lower portion of cylinder flange 213 and movable holding members 122 of one-third arc form for holding the upper portion of cylinder flange 213 from the left and right that are arranged in a freely opening and closing state above both ends of stationary holding member 121.

As shown in FIG. 4 and FIG. 7, piston-driving mechanism 130 of liquid injector 100 of the present embodiment includes, as the slider member, slide rod 131 that is slender in the forward and rearward direction, and this slide rod 131 is supported by head main unit 113 in a state that prevents rotation around its axis but that allows free forward and rearward sliding.

Piston-pressing member 132 is formed as a single unit at the forward end of slide rod 131, and a pair of elastically opening and closing engaging catches 133 are installed on the right and left sides of this piston-pressing member 132. Threaded hole 134 is formed that extends forward from the center of the rear surface of slide rod 131, and external threads 136 that engage with this threaded hole 134 are formed on screw shaft 137, which is the rotation member.

This screw shaft 137 is supported in the diametrical direction by head main unit 113 by means of radial bearing 138 in a state that allows free rotation, and is supported in the axial direction by thrust bearing 139 that allows free rotation. More specifically, ring-shaped flange 141 is formed in the vicinity of the rearward end of screw shaft 137, and the rear surface of this flange 141 butts against ring-shaped thrust bearing 139.

Load cell 142, which is the stress detection means and which has the same shape as thrust bearing 139, contacts the rear surface of this thrust bearing 139. This load cell 142 is fixed to head main unit 113, but thrust-bearing 139 is supported by head main unit 113 in a state that allows minute displacement in the forward and rearward directions.

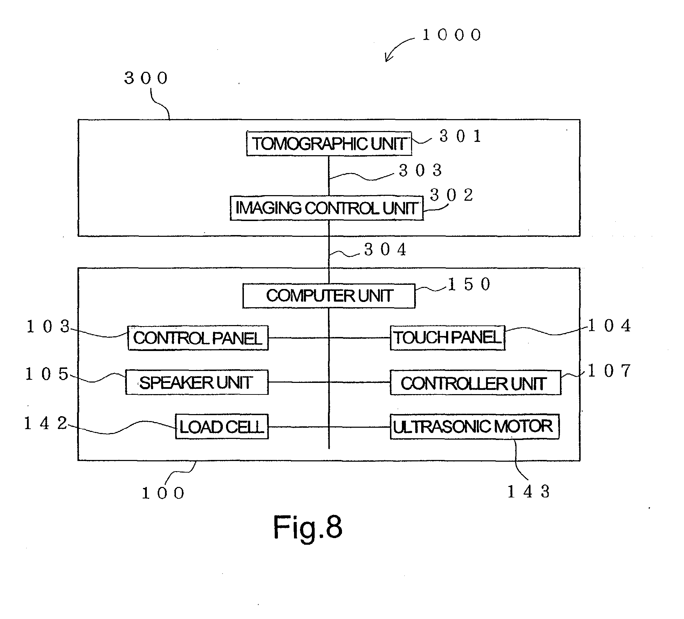

As shown in FIG. 8, piston-driving mechanism 130 includes ultrasonic motor 143 as a drive motor, and as shown in FIG. 4, this ultrasonic motor 143 is linked to screw shaft 137 by belt structure 144. More specifically, belt pulley 145 is installed as a single unit at the rearward end of screw shaft 137, a belt pulley (not shown) is also installed as a single unit to the rotor of ultrasonic motor 143, and endless belt 146 is installed on these belt pulleys 145.

In injection head 110 of liquid injector 100 of the present embodiment, each of the parts is formed of nonmagnetic materials, and parts that cannot be formed from nonmagnetic material are demagnetized. For example, ultrasonic motor 143 is formed of nonmagnetic metals such as phosphor bronze alloy (Cu+Sn+P), titanium alloy (Ti-6AI-4V), and magnesium alloy (Mg+AI+Zn) and thus does not generate a magnetic field during operation. Components such as load cell 142 and screw shaft 137 are similarly formed of nonmagnetic metals, and components such as belt structure 144 and head main unit 113 are formed from a nonmagnetic resin.

As shown in FIG. 8, each of the parts of liquid injector 100 of the present embodiment such as ultrasonic motor 143 and load cell 142 are connected to computer unit 150, and through the implementation of integrated control over each of the parts in accordance with a computer program that is installed in this computer unit 150, the various means such as a type input means, pressure detection means, pressure storage means, state detection means, drive control means, and pressure display means are logically realized as various functions (not shown).

Although the details will be described later, various types of liquid syringes 200 can be interchangeably loaded in liquid injector 100, and as an example, the liquid name or viscosity and the capacity or inside diameter of cylinder 210 may be registered as data for each item of identification data of these liquid syringes 200. Then, when input operations are performed at control panel 103 for the identification data of liquid syringe 200, computer unit 150 reads the corresponding registered data and sets the operation mode.

When computer unit 150 causes piston-driving mechanism 130 to operate and causes piston 220 to be pressed into cylinder 210, computer unit 150 calculates the injection pressure of the liquid based on the stress detected by load cell 142 and the registered data of liquid syringe 200. Still further, computer unit 150 implements feedback-control over piston-driving mechanism 130 in accordance with this injection pressure and simultaneously provides real-time display output of the injection pressure on touch panel 104.

Computer unit 150 also stores data of injection pressures for each of the various states of piston-driving mechanism 130 and liquid syringe 200, and by collating the injection pressures of these stored data with the detected injection pressure, detects data of the state of piston-driving mechanism 130 and liquid syringe 200 and thus controls the operation of piston-driving mechanism 130 in accordance with the detected states.

More specifically, computer unit 150 stores data for the injection pressures for the state in which liquid is normally injected from cylinder 210 into a patient, the injection pressures for a state in which air is mixed with the liquid, and the injection pressures for a state in which air is injected into the patient from cylinder 210; and as a result, computer unit 150 forcibly halts piston-driving mechanism 130 when data are not detected for a state of normal injection of liquid, when data are detected for a state in which air is mixed in the liquid, or when data are detected for a state in which air is being injected.

Finally, when controlling the operations of piston-driving mechanism 130 as described hereinabove, computer unit 150 of liquid injector 100 exchanges various data with injection control unit 101 of MRI device 300 and thus directs various operations in which liquid injector 100 and MRI device 300 cooperate.

### The Working of the Embodiment

In the above-described configuration, tomographic images can be taken from a patient by means of MRI device 300 in the liquid injection system 1000 of the present embodiment, and the patient can be injected with a liquid such as a contrast medium.

In this case, as shown in FIG. 7, liquid syringe 200 is linked to the patient by means of, for example, an extension tube, and this liquid syringe 200 is placed in injection head 110 of liquid injector 100. At this time, cylinder 210 of liquid syringe 200 is held by cylinder-holding structure 120 and piston 220 is held by piston-driving mechanism 130.

The operator then performs the input operation for the identification data of liquid syringe 200 in control panel 103 of liquid injector 100, whereupon liquid injector 100, in response to this input operation, reads the various types of data such as the capacity or inner diameter of cylinder 210 and sets the operation modes.

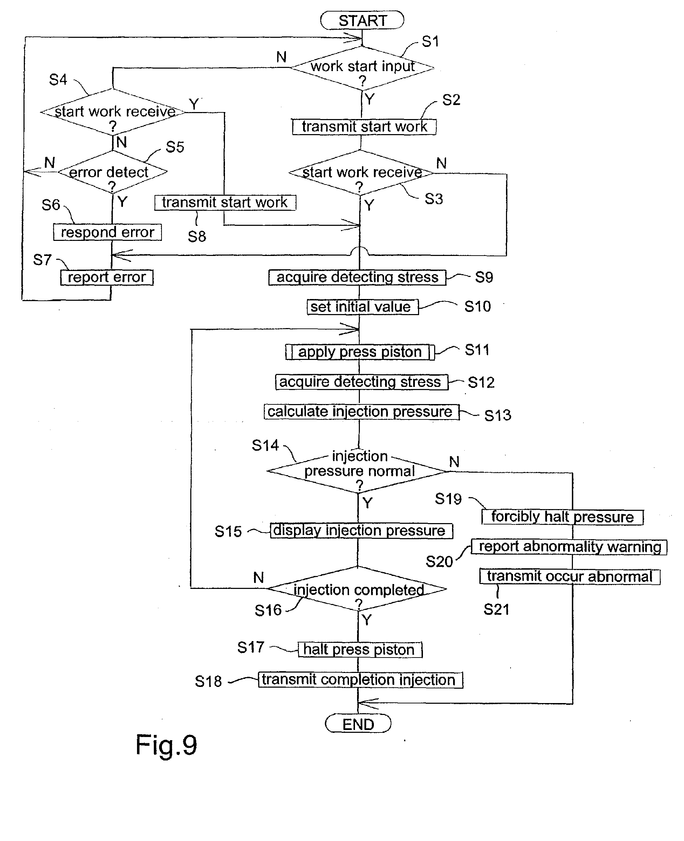

In this state, the operator performs an input operation at control panel 103 of liquid injector 100 of the starting work, whereupon, as shown in FIG. 9, liquid injector 100 detects this input (Step S1), and transmits data of the starting work to MRI device 300 (Step S2).

If a data reply indicating the start of work from MRI device 300 (Step S3) is received in response to the above-described data transmission, liquid injector 100 begins a series of work operations (Step S9-), but if data for the start of work is not received or if data of error guidance is received (Step S3), an error guidance message such as "An XX error has occurred. Please check the MRI device" is reported on touch panel 104 or by speaker unit 105 and the process returns to the initial state (Step S7).

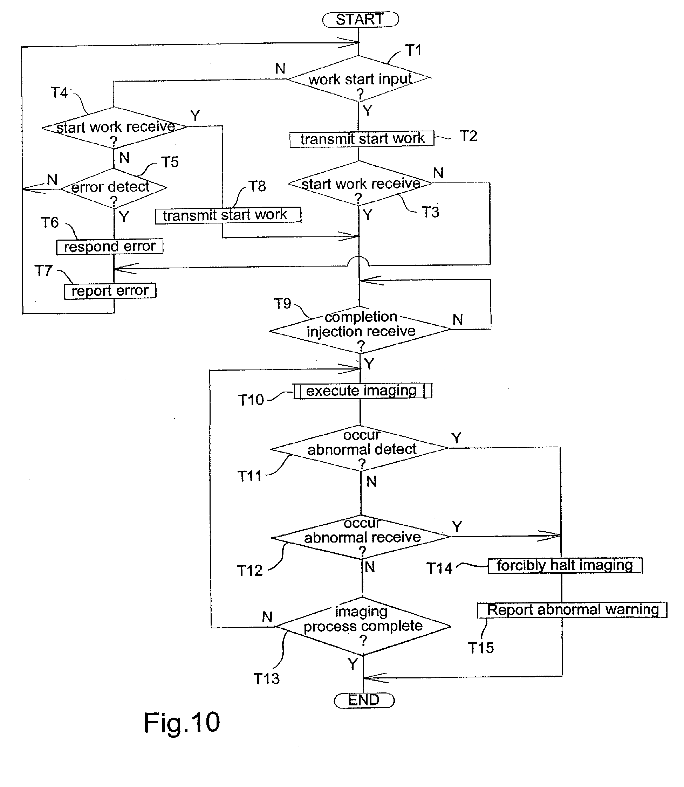

As shown in FIG. 10, when MRI device 300, which is in a state in which an input operation indicating the start of work has not been performed in imaging control unit 302, receives data indicating the start of work from liquid injector 100 (Step T4) as described in the foregoing explanation, MRI device 300 diagnoses its own state (Step T5), and if no error is detected in this diagnosis, responds to liquid injector 100 with data indicating the start of work (Step T8).

If MRI device 300 detects an error in the above-described self-diagnosis (Step T5), MRI device 300 responds with data giving error guidance to liquid injector 100 (Step T6), reports an error guidance message such as "An XX error has occurred. Please check ---", and returns to the initial state (Step T7).

Even if the operator should first perform an input operation for the start of work at MRI device 300 instead of at liquid injector 100, the start of work is mutually confirmed by means of intercommunication between MRI device 300 and liquid injector 100 as in the above-described case and as shown in FIG. 10 and FIG. 9 (Steps T1-T3 and Steps S4-S8).

Then, as shown in FIG. 9, computer unit 150 in liquid injector 100 that has confirmed the start of work acquires the electrical resistance from load cell 142 as an electrical signal without causing ultrasonic motor 143 of piston-driving mechanism 130 to operate (Step S9) and executes initial settings with this electrical resistance indicating "0" stress (Step S10).

Computer unit 150 next causes ultrasonic motor 143 of piston-driving mechanism 130 to operate, and thus causes piston 220 to be pressed into cylinder 210 of liquid syringe 200 (Step S11).

At this time, as shown in FIG. 4, screw shaft 137 that is linked to ultrasonic motor 143 by belt structure 144 is subjected to rotational drive, and because this screw shaft 137 is supported in the axial direction by way of thrust bearing 139 by load cell 142 that is fixed to head main unit 113, slide rod 131 that is linked by the thread structure to screw shaft 137 is caused to slide.

This slide rod 131 therefore presses piston 220 into cylinder 210, and liquid is thus injected from this cylinder 210 into the patient. At this time, the stress that acts upon slide rod 131 that applies pressure against piston 220 also acts upon screw shaft 137, and the stress that acts upon this screw shaft 137 therefore acts upon load cell 142 by way of thrust bearing 139.

Here, as shown in FIG. 9, computer unit 150 acquires the stress that is detected by load cell 142 during the execution of liquid injection as previously described (Step S12), and calculates the injection pressure based on the detected stress that corresponds to the viscosity of the liquid and the inner diameter of cylinder 210 that were initially set (Step S13).

Computer unit 150 then verifies whether this injection pressure is normal (Step S14), and if normal, causes the injection pressure to be displayed on touch panel 104 (Step S15). The above operations are repeated (Steps S11-S16) until the completion of injection is detected based on, for example, a comparison of the operating distance of piston-driving mechanism 130 and the effective distance of piston 220 (Step S16).

Since data for the optimum injection pressure has been set by means of the initial setting of the operating mode, when the injection of liquid is executed as described above (Steps S11-S16), the output of ultrasonic motor 143 is subjected to feedback control such that the calculated injection pressure matches the optimum pressure that has been set (Step S11).

When the liquid injection has been completed (Step S16), the operation of ultrasonic motor 143 is forcibly halted (Step S17) and data indicating the completion of injection are transmitted from computer unit 150 of liquid injector 100 to imaging control unit 302 of MRI device 300 (Step S18).

When the calculated injection pressure is not normal (Step S14) in liquid injector 100, the operation of ultrasonic motor 143 is forcibly halted (Step S19). More specifically, computer unit 150 stores data of the injection pressures for a state in which liquid is being injected normally from cylinder 210 to the patient, the injection pressures for a state in which air is mixed with the liquid, and the injection pressures for a state in which air is being injected from cylinder 210 to the patient; and thus forcibly halts piston-driving mechanism 130 when data are not detected for the state in which liquid is being normally injected, when data are detected for a state in which air is mixed with the liquid, or when data are detected for a state in which air is being injected.

Warnings of abnormalities such as "Abnormal pressure has been detected-Please check the syringe and tube", "Mixed air has been detected-Please check the cylinder and tube"; and "Air injection has been detected-Please check the patient" are reported by display output on touch panel 104 or speech output on speaker unit 105 (Step S20), and data for the occurrence of these abnormalities are transmitted from computer unit 150 to imaging control unit 302 (Step S21).

Then, as shown in FIG. 10, MRI device 300 that has received data indicating the completion of injection from liquid injector 100 (Step T9) takes tomographic images from the patient that has been injected with the contrast medium (Steps T10 and T13). MRI device 300, upon detection of the occurrence of an abnormality (Step T11) or upon receiving data from liquid injector 100 indicating the occurrence of an abnormality (Step T12), forcibly halts the imaging operation and reports the occurrence of the abnormality to the operator (Steps T14 and T15).

### The Effect of the Embodiment

In liquid injection system 1000 of the present invention, liquid can be injected from liquid syringe 200 to the patient because liquid injector 100 holds cylinder 210 of liquid syringe 200 by cylinder-holding structure 120 and applies pressure against piston 220 by means of piston-driving mechanism 130 as described in the foregoing explanation. In addition, the injection operation can be appropriately controlled because the stress of the pressure applied against piston 220 is detected by load cell 142.

Moreover, piston-driving mechanism 130 is supported by head main unit 113 in a state that allows free displacement, and load cell 142 that detects the stress that acts upon this piston-driving mechanism 130 is fixed to head main unit 113. Since load cell 142 is therefore not arranged on the movable portion of piston-driving mechanism 130, the device features a simple configuration, high productivity, and excellent reliability.

In particular, slide rod 131 that applies pressure against piston 220 in piston-driving mechanism 130 is supported in a state that allows free sliding but that prevents rotation, and load cell 142 detects the stress that acts in the axial direction upon screw shaft 137 that can rotate freely but that cannot slide, and further, that is linked to slide rod 131 by a screw structure. As a result, load cell 142 has a simple construction and can reliably detect the stress that acts upon piston 220.

Still further, screw shaft 137 is both supported in the diametrical direction by radial bearing 138 and supported in the axial direction by thrust bearing 139, whereby screw shaft 137 has a simple structure and can rotate freely. The stress that acts in the axial direction upon this thrust bearing 139 is detected by load cell 142, and this load cell 142 can therefore detect the stress that acts upon piston 220 without impeding the rotation of screw shaft 137.

The linking of ultrasonic motor 143 and screw shaft 137 by belt structure 144 not only allows displacement of screw shaft 137 in the axial direction without impeding the transmission of power, but also allows the detection of the stress that acts upon screw shaft 137 to be reliably detected by load cell 142.

A plurality of types of liquid syringe 200 are interchangeably loaded in liquid injector 100, and variations in the viscosity of the liquid or in the inner diameter of cylinder 210 may therefore result in different injection pressures even when the stress detected by load cell 142 is the same. In liquid injector 100 of the present embodiment, however, computer unit 150 registers the various types of data for liquid syringes 200 according to each type of identification data and reads the various types of data when identification data are received as input, whereby the injection pressure can be calculated appropriately based on the stress detected by load cell 142.

Computer unit 150 then subjects piston-driving mechanism 130 to feedback control that accords with this injection pressure, and liquid can therefore be injected to the patient at the appropriate pressure. The above-described injection pressure is then displayed as data on touch panel 104 in real time, and the operator is therefore always able to verify the injection pressure.

In addition, computer unit 150 stores data for pressures detected by computer unit 150 for each of the various states of piston-driving mechanism 130 and liquid syringe 200, collates these stored pressures with the detected pressures to detect data for the states of piston-driving mechanism 130 and liquid syringe 200, and then controls the operations of piston-driving mechanism 130 in accordance with these detected states, whereby computer unit 150 is able to control liquid injection precisely.

More specifically, computer unit 150 stores data of injection pressures for the state in which liquid is injected normally from cylinder 210 to a patient, and forcibly halts piston-driving mechanism 130 when data are not detected for a state in which the liquid is being injected normally, whereby mistakes in medical treatment in which liquid is injected into a patient at an abnormal pressure can be prevented.

Still further, computer unit 150 stores data of the injection pressures for a state in which air is mixed in the liquid as well as the injection pressures for a state in which air is being injected from cylinder 210 to the patient, and as a result, forcibly halts the piston-driving mechanism 130 upon detecting data for a state in which air is mixed in the liquid or upon detecting data for a state in which air is being injected, whereby mistakes in medical treatment in which air is injected to the patient can be prevented.

When the above-described states have been detected, warnings of abnormality are reported by display output on touch panel 104 or speech output on speaker unit 105, and the operator can swiftly recognize and take proper measures for the occurrence of the abnormality.

Still further, liquid injector 100 and MRI device 300 are caused to operate in concert and perform various operations in unison, whereby MRI device 300 is able to take tomographic images at the proper timings for the injection of the contrast medium, and liquid injector 100 is able to inject liquids such as a contrast medium from liquid syringe 200 at the proper timings for the taking of tomographic images. As a result, in liquid injection system 1000 of the present embodiment, a single operator working by himself or herself is able to handle the proper operation of liquid injector 100 and MRI device 300, and a reduction in costs for personnel can thus be obtained.

When liquid injector 100 detects the occurrence of an abnormality as described above, data for this occurrence of an abnormality are transmitted to MRI device 300 and the MRI device 300 also swiftly forcibly halts imaging operations, whereby the operator is able to swiftly take proper measures for the occurrence of an abnormality without being impeded by the MRI device 300.

When data of the occurrence of an abnormality are transmitted from liquid injector 100 to MRI device 300, MRI device 300 also reports the occurrence of the abnormality to the operator, whereby an operator that is working alone can be swiftly informed of and recognize the occurrence of an abnormality in liquid injector 100 even when the operator has moved from the position of liquid injector 100 to the position of MRI device 300.

Finally, liquid injector 100 of the present embodiment includes as its driving source ultrasonic motor 143 that is formed of nonmagnetic material and that does not generate an unnecessary magnetic field during operation, and each component such as load cell 142 is also formed of nonmagnetic materials, and liquid injector 100 can therefore be used without problems in the vicinity of MRI device 300.

### Modifications of the Embodiment

The present invention is not in any way limited to the above-described embodiment, and allows various modifications within the scope of the invention. For example, a case was described in the above-described embodiment in which liquid injection system 1000 included MRI device 300 as an imaging diagnostic apparatus, but a CT scanner, a PET device, an ultrasonic diagnostic device, an angio device, or an MRA apparatus may be used as the imaging diagnostic apparatus.

Although a case was described in the above-described embodiment in which various types of data of liquid syringes 200 are registered in liquid injector 100 in advance and in which liquid injector 100 controls the injection operation in accordance with this registered data; the various types of data of a plurality of types of liquid syringes 200 may be registered in liquid injector 100 in advance, and the operator may then perform input operations of the various types of data of liquid syringes 200 in liquid injector 100 with each instance of use.

In such a case, the operator may also perform input of numerical values for the filled capacity by the manual manipulation of control panel 103 or touch panel 104, or liquid syringes 200 may be given barcode marks in which data such as the filled capacity or cylinder inner diameter have been registered and liquid injector 100 may then use a barcode reader (not shown) to scan and read these barcode marks.

Although a case was described in the above-described embodiment in which threaded hole 134 is formed in slide rod 131 and external threads 136 are formed on screw shaft 137, but it is also possible for external threads 136 to be formed on slide rod 131 and threaded hole 134 to be formed in screw shaft 137 (not shown).

Although a case was described in the above-described embodiment in which external threads 136 of screw shaft 137 directly engage threaded hole 134 of slide rod 131, a ball screw structure (not shown) may be formed in which this threaded hole 134 and external threads 136 engage by way of a ball bearing.

Although a case was described in the above-described embodiment in which ultrasonic motor 143 is linked to screw shaft 137 by means of belt structure 144 that allows free displacement in the axial direction, it is also possible, for example, to directly connect screw shaft 137 and ultrasonic motor 143 and support this ultrasonic motor 143 in a state that prevents rotation while allowing displacement in the axial direction together with screw shaft 137 (not shown).

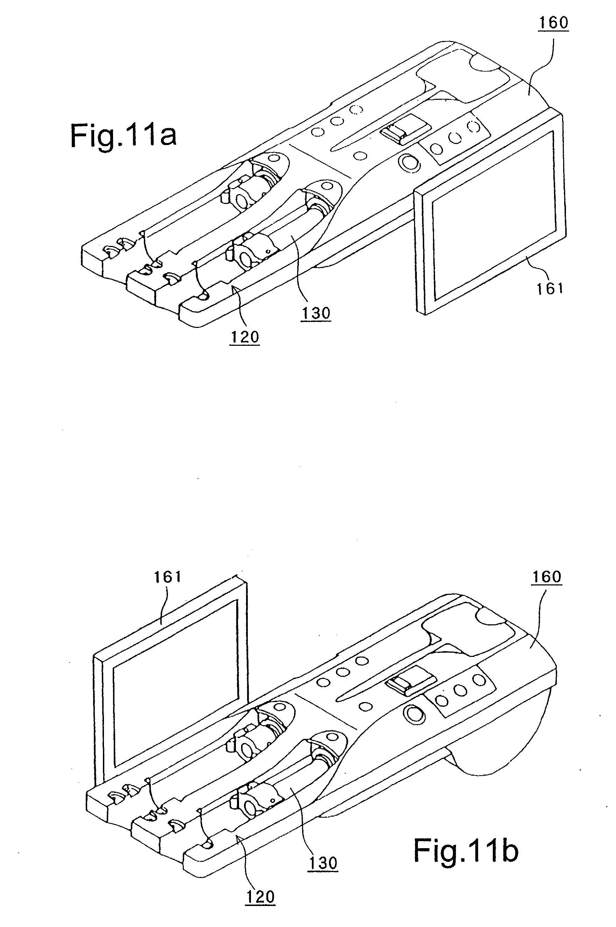

A case was described in the above-described embodiment in which only one liquid syringe 200 is loaded in injection head 110 that has one cylinder-holding structure 120 and one piston-driving mechanism 130, but, as shown in FIG. 11, a plurality of liquid syringes 200 may also be loaded in injection head 160 having a plurality of each of cylinder-holding structures 120 and piston-driving mechanisms 130.

Although a case was described in the above-described embodiment in which touch panel 104 that displays various types of data such as injection pressure is mounted on imaging control unit 101 that is separate from injection head 110, as shown in FIG. 11, this type of display panel 161 may also be provided in juxtaposition with injection head 160. In such a case, various types of data are displayed in the vicinity of the position of loading liquid syringe 200 and the operator can therefore more closely monitor these data.

Finally, a case was described in the above-described embodiment in which the various means were realized logically as various functions of liquid injector 100 through operations in accordance with a computer program that is installed in computer unit 150. However, each of these various means may be formed as individual hardware components, or a portion may be stored as software in, for example, computer unit 150 and a portion may be formed as hardware components.

While a preferred embodiment of the present invention has been described using specific terms, such description is for illustrative purposes only, and it is to be understood that changes and variations may be made without departing from the spirit or scope of the following claims.

## Claims

1. A liquid injector 100 wherein a piston 220 is freely slidably inserted into a cylinder 210 that is filled with a liquid and wherein said cylinder 210 and said piston 220 are moved relative to each other to thus inject said liquid into a patient; said liquid injector 100 comprising:
a cylinder-holding structure 120 for detachably holding said cylinder 210;
a piston-driving mechanism 130 for applying pressure to at least said piston 220 to press said piston 220 into said cylinder 210;
an injector main body 110 to which said cylinder-holding structure 120 is fixed and which supports said piston-driving mechanism 130 displaceably in the direction of sliding of said piston 220; and
a stress detection means 142 that is fixed to this injector main body 110 for detecting stress that acts upon said piston-driving mechanism 130 that applies pressure against said piston 220.

2. A liquid injector according to claim 1, wherein said piston-driving mechanism comprises:
a slider member in which a threaded hole is formed and which is supported by said injector main body at a position that contacts said piston and in a state that allows free sliding but that prevents rotation;
a rotation member having external threads formed on the outer surface that engage with the threaded hole of this slider member and being supported by said injector main body in a state that allows free rotation but that prevents sliding; and
a drive motor for driving this rotation member;
wherein said stress detection means detects stress that acts upon said rotation member.

3. A liquid injector according to claim 1, wherein said piston-driving mechanism comprises:
a slider member having external threads on the outer surface and being supported by said injector main body at a position that contacts said piston and in a state that allows free sliding but that prevents rotation;
a rotation member in which a threaded hole is formed that engages with the thread shape of the slider member and being supported by said injector main body in a state that allows free rotation but that prevents sliding; and
a drive motor for driving this rotation member;
wherein said stress detection means detects stress that acts upon said rotation member.

4. A liquid injector according to claim 2, wherein:
said piston-driving mechanism includes a bearing mechanism for supporting said rotation member in the diametrical direction and in the axial direction; and
said stress detection means detects stress that acts upon said bearing mechanism in said axial direction.

5. A liquid injector according to claim 4, wherein said bearing mechanism is made up of:
a radial bearing for supporting said rotation member in the diametrical direction; and
a thrust bearing for supporting said rotation member in the axial direction;
wherein said stress detection means detects stress that acts upon said thrust bearing.

6. A liquid injector according to any one of claims 1 to 5, wherein said drive motor and said rotation member are linked by a belt mechanism composed of at least one pair of belt pulleys and at least one endless belt.

7. A liquid injector according to any one of claims 1 to 5, wherein said stress detection means is composed of a load cell that is formed of nonmagnetic materials.

8. A liquid injector according to any one of claims 1 to 5, further comprising a drive control means for exercising feedback control of said piston-driving mechanism in accordance with stress detected by said stress detection means.

9. A liquid injector according to any one of claims 1 to 5, further comprising a pressure detection means for calculating injection pressure of said liquid based on stress that is detected by said stress detection means.

10. A liquid injector according to claim 9, wherein:
a plurality of types of said liquid syringes are interchangeably loaded in said cylinder-holding structure;
said liquid injector further comprises a type input means for receiving input of identification data of said liquid syringe that is held by said cylinder-holding structure; and
said pressure detection means detects said injection pressure based on stress detected by said stress detection means in accordance with said identification data of the liquid syringe that have been received as input.

11. A liquid injector according to claim 9, further comprising a pressure display means for displaying in real time the pressure detected by said pressure detection means.

12. A liquid injector according to claim 11, wherein said pressure display means comprises a display panel that is arranged in a position adjacent to at least one of said cylinder-holding structure and said piston-driving mechanism.

13. A liquid injector according to claim 9, further comprising:
a pressure storage means for storing as data pressures that are detected by said pressure detection means for each of the various states of said piston-driving mechanism and said liquid syringe; and
a state detection means for collating said pressures that have been stored as data with said pressures that are detected to detect data of the states of said piston-driving mechanism and said liquid syringe;
wherein said drive control means controls the operations of said piston-driving mechanism in accordance with said states that are data-detected by said state detection means.

14. A liquid injector according to claim 13, wherein:
said pressure storage means stores as data said pressures of states in which liquid is injected normally from said cylinder to said patient; and
said drive control means forcibly halts said piston-driving mechanism when a state in which said liquid is being normally injected is not detected.

15. A liquid injector according to claim 13, wherein:
said pressure storage means stores as data said pressures of states in which air is being injected from said cylinder to said patient; and said drive control means forcibly halts said piston-driving mechanism when said state in which air is being injected is detected.

16. A liquid injector according to claim 13, wherein:
said pressure storage means stores as data said pressures of states in which air is mixed with said liquid; and
said drive control means forcibly halts said piston-driving mechanism when said state in which said air is mixed with said liquid is detected.
